(19) **Europäisches Patentamt**

**European Patent Office**

**Office européen des brevets**

(11) **EP 0 615 436 B1**

(12) **EUROPÄISCHE PATENTSCHRIFT**

(45) Veröffentlichungstag und Bekanntmachung des Hinweises auf die Patenterteilung:
**10.04.1996 Patentblatt 1996/15**

(21) Anmeldenummer: **93917760.6**

(22) Anmeldetag: **06.08.1993**

(51) Int. Cl.[6]: **A61K 7/50**, A61K 7/06

(86) Internationale Anmeldenummer: **PCT/EP93/02094**

(87) Internationale Veröffentlichungsnummer:
**WO 94/07464 (14.04.1994 Gazette 1994/09)**

(54) **HAARKURMITTEL**

HAIR TREATMENT AGENT

AGENT DE TRAITEMENT CAPILLAIRE

(84) Benannte Vertragsstaaten:
**DE ES FR GB IT**

(30) Priorität: **05.10.1992 DE 4233353**

(43) Veröffentlichungstag der Anmeldung:
**21.09.1994 Patentblatt 1994/38**

(73) Patentinhaber: **Wella Aktiengesellschaft**
**D-64274 Darmstadt (DE)**

(72) Erfinder:
- **AEBY, Johann**
  **CH-1723 Marly (CH)**
- **MAGER, Herbert**
  **CH-1723 Marly (CH)**
- **CLAUSEN, Thomas**
  **D-64665 Alsbach (DE)**
- **HOCH, Dieter**
  **D-64319 Pfungstadt-Eich (DE)**

(56) Entgegenhaltungen:
**EP-A- 0 366 070          DE-A- 3 301 667**

**Beschreibung**

Gegenstand der Erfindung ist ein Haarkurmittel, das eine Kombination aus einem oxethylierten Fettalkohol, einem oxethylierten Glycerylfettsäureester, einer organischen Säure und einem kationischen Cellulosederivat auf der Basis einer wäßrig-alkoholischen Zubereitung enthält.

Die Haare werden durch Einwirkungen verschiedener Art in ihren physikalischen, chemischen und morphologischen Eigenschaften negativ beeinflußt. So wird durch kosmetische Behandlungen, wie wiederholtes Bleichen, Dauerwellen und Färben, aber auch durch häufiges Waschen der Haare mit entfettenden Tensiden sowie durch Klimaeinflüsse wie Feuchte- und Temperaturunterschiede oder die intensive Einwirkung von Sonnenlicht die Haarstruktur geschädigt. Das Haar wird spröde und verliert seinen Glanz. Das derart geschädigte Haar lädt sich beim Kämmen und Bürsten elektrostatisch auf, und die aufgerauhte Haaroberfläche führt durch die Bildung von Verfilzungen und Verknotungen zu einer schlechten Kämmbarkeit und Entwirrbarkeit des Haares. Haarkurmittel mit einer pflegenden und kämmbarkeitsverbessernden Wirkung haben daher erhebliche Bedeutung erlangt.

Derartige Mittel werden beispielsweise häufig in Form einer klaren Haarpflegespülung oder auch in Emulsionsform, als sogenannte Creme-Rinses, nach der Haarwäsche im noch nassen Haar verteilt, einige Minuten bis eine Stunde einwirken gelassen und dann mit Wasser ausgespült.

Als Wirkstoffe zur Verbesserung der Haarstruktur werden hauptsächlich kationische Tenside, insbesondere quaternäre Ammoniumverbindungen, wie Cetyltrimethylammoniumchlorid, in Kombination mit verschiedenen wachsartigen Zusätzen, wie zum Beispiel Vaseline, Fettalkoholen und Fettsäureestern, eingesetzt.

Haarkurmittel auf der Basis der vorstehenden konditionierenden Wirkstoffe zeigen jedoch nur bei der Behandlung von trockenem und porösem Haar zufriedenstellende Ergebnisse. Für die Behandlung von rasch nachfettendem Haar sind sie weniger gut geeignet, da durch ihre Anwendung die natürliche Nachfettung der Haare noch verstärkt wird, wodurch sich wiederum die Haltbarkeit der Frisur verschlechtert.

Die Ursachen der starken Nachfettung der Haare sind zum einen die in diesen Mitteln enthaltenen kationischen Tenside, zum anderen die nach dem Ausspülen im Haar verbleibenden Reste des Haarkurmittels. Die vom Haar absorbierten kationischen Tenside bewirken eine Hydrophobierung der Haaroberfläche, wodurch sich die Absonderungen der Talgdrüsen rascher im Haar verteilen können.

Die in üblichen Haarkurmitteln enthaltenen kationischen Tenside haben weiterhin den Nachteil, daß sie in höheren Konzentrationen schlecht hautverträglich und biologisch schwer abbaubar sind.

Es sind bereits Haarkurmittel beschrieben worden, die statt kationischer Tenside kationische Cellulosederivate enthalten.

Aus der JP-OS 65-193 717 sind schäumende Haarbehandlungsmittel, die unter anderem als Haarspülung oder Haarcreme vorliegen können, bekannt, welche einen Gehalt an einem kationischen Cellulosederivat, nicht-ionischen Tensiden, Zitronensäure, Ethanol und Wasser aufweisen. Die beschriebenen Haarbehandlungsmittel sind zwar frei von kationischen Tensiden, sie sind jedoch in bezug auf den Pflegeeffekt und die Kämmbarkeitsverbesserung bei Anwendung auf längerem, porösem Haar nicht zufriedenstellend.

Demgemäß bestand die Aufgabe, ein von kationischen Tensiden freies Haarkurmittel mit guten haarkonditionierenden Eigenschaften zur Verfügung zu stellen, dessen Inhaltsstoffe gut hautverträglich und biologisch leicht abbaubar sind und dem Haar eine gute Frisierbarkeit und einen guten Halt verleihen sollen.

Es wurde nunmehr gefunden, daß diese Aufgabe durch ein Haarkurmittel, dadurch gekennzeichnet, daß es eine Kombination aus den Komponenten

(A) 0,2 bis 10,0     Gewichtsprozent mindestens eines mit 1 bis 6 Mol Ethylenoxid oxethylierten $C_{12}$- bis $C_{20}$-Fettalkohols,

(B) 0,2 bis 10,0     Gewichtsprozent mindestens eines mit 4 bis 10 Mol Ethylenoxid oxethylierten Glycerylfettsäureesters einer Fettsäure mit 12 bis 20 Kohlenstoffatomen,

(C) 0,01 bis 3,0     Gewichtsprozent mindestens eines kationischen Cellulosederivates,

(D) 0,01 bis 2,0     Gewichtsprozent mindestens einer wasserlöslichen, physiologisch verträglichen organischen Säure mit 1 bis 10 Kohlenstoffatomen,

(E) 1,0 bis 40,0     Gewichtsprozent Ethanol und/oder Isopropanol,

und

(F) 50 bis 80     Gewichtsprozent Wasser

enthält und frei von kationischen Tensiden ist, in hervorragender Weise erfüllt wird.

Das erfindungsgemäße Haarkurmittel enthält bevorzugt 0,5 bis 7 Gewichtsprozent der Komponente (A). Als bevorzugte Fettalkoholoxethylate der Komponente (A) sind die des Laurylalkohols mit einem Oxethylierungsgrad von 2 bis 4 in dem erfindungsgemäßen Mittel enthalten.

Das erfindungsgemäße Mittel enthält als Komponente (B) bevorzugt 0,5 bis 7 Gewichtsprozent des mit 4 bis 10 Mol Ethylenoxid oxethylierter Glycerylfettsäureesters einer Fettsäure mit 12 bis 20 Kohlenstoffatomen, wobei die oxethylierten Glycerylester der Kokosfettsäure und die oxethylierten Glycerylmonoester der Laurinsäure bevorzugt sind. Besonders bevorzugt wird mit 7 Mol Ethylenoxid oxethylierter Glycerinkokosfettsäureester eingesetzt (zum Beispiel Cetiol®HE der Firma Henkel, Düsseldorf, Deutschland).

Das neue Haarkurmittel enthält die Komponente (C) bevorzugt in einer Menge von 0,1 bis 2 Gewichtsprozent. Als kationisches Cellulosederivat kommen insbesondere solche in Betracht, wie sie in der US-PS 3 472 840 beschrieben sind und im Handel unter der Bezeichnung Polymer JR® (Union Carbide, New York, USA) erhältlich sind, wobei das Handelsprodukt Polymer JR® 400 bevorzugt ist.

Die organische Säure Komponente (D) kann beispielsweise ausgewählt sein aus Benzoesäure, Salicylsäure, Glutarsäure, Pimelinsäure, Äpfelsäure, Adipinsäure, Bernsteinsäure, Weinsäure, Zitronensäure oder Milchsäure, wobei Zitronensäure und Milchsäure bevorzugt sind. Das erfindungsgemäße Mittel enthält bevorzugt 0,1 bis 1 Gewichtsprozent der Komponente (D).

Das Haarkurmittel enthält als Komponente (E) bevorzugt 5 bis 35 Gewichtsprozent Ethanol und/oder Isopropanol.

Das erfindungsgemäße Mittel enthält das Wasser in einer bevorzugten Menge von 55 bis 75 Gewichtsprozent.

Das Haarkurmittel kann bis zu 1 Gew. % Silicone enthalten, es ist jedoch vorzugsweise frei von Siliconen und anderen schwer abbaubaren Substanzen.

Das Haarkurmittel weist vorzugsweise einen pH-Wert von 1 bis 6, besonders bevorzugt von 1,5 bis 4, auf.

Das Haarkurmittel gemäß der vorliegenden Erfindung kann, mit Ausnahme von kationischen Tensiden, zusätzlich alle diejenigen Bestandteile enthalten, die in Haarkurmitteln üblicherweise eingesetzt werden, insbesondere anionische oder nichtionische Tenside, beispielsweise Alkali-, Erdalkali-, Ammonium-oder Alkanolaminsalze von Alkylsulfonaten, Alkylsulfaten und Alkylethersulfaten, oxethylierte Fettalkohole, Fettsäurealkanolamide und oxethylierte Sorbitanfettsäureester, in einer Menge von 0,01 bis 3 Gewichtsprozent; Schaumsynergisten; Schaumstabilisatoren; Sequestriermittel; Emulgatoren; Naturstoffe; Pigmente; Parfümöle in einer Menge von 0,5 bis 5,0 Gewichtsprozent; Trübungsmittel, wie zum Beispiel Ethylenglykoldistearat, in einer Menge von etwa 0,5 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Verdickungsmittel, wie beispielsweise Kokosfettsäurediethanolamid oder Hydroxyalkylcellulose, in einer Menge von 0,5 bis 10,0 Gewichtsprozent; niedere aliphatische Alkohole mit 1 bis 4 Kohlenstoffatomen in einer Menge von 5 bis 80 Gewichtsprozent; Puffersubstanzen, wie beispielsweise Natriumcitrat oder Natriumphosphat, in einer Menge von 0,1 bis 1,0 Gewichtsprozent; Lösungsvermittler, wie zum Beispiel oxethyliertes, gegebenenfalls hydriertes Rizinusöl, in einer Menge von 0,1 bis 1,0 Gewichtsprozent; sowie Farbstoffe, wie zum Beispiel Fluorescein-Natriumsalz, in einer Menge von 0,1 bis 1,0 Gewichtsprozent; weiterhin haarpflegende Zusätze, wie zum Beispiel Fettsäureester, Fettalkohole, Fettsäureglyceride, ethoxylierte oder propoxylierte gesättigte Fettalkohole; natürliche, modifizierte natürliche oder synthetische Polymere, wie zum Beispiel anionische oder nichtionische Cellulosederivate, Chitosan, kationische Chitin- oder Chitosanderivate, Polyvinylpyrrolidon oder Polymerisate von Acrylsäure und/ oder deren Derivaten; Pflegestoffe, wie zum Beispiel Lanolinderivate, Cholesterin und Pantothensäure, in einer Menge von 0,1 bis 10 Gewichtsprozent; außerdem physiologisch verträgliche anorganische Salze, wie zum Beispiel Natriumchlorid und Natriumsulfat; sowie ferner Feuchthaltemittel; Lichtschutzmittel; Antioxidantien; Komplexbildner; Antischuppenwirkstoffe; kosmetische Öle und Wachse sowie Konservierungsstoffe.

Während bei den bisher beschriebenen Haarbehandlungsmitteln, mit einem Gehalt an einem kationischen Cellulosederivat, keine zufriedenstellenden haarkonditionierenden Eigenschaften festzustellen sind, zeigt das erfindungsgemäße Haarkurmittel auf der Basis einer synergistischen Kombination der Komponenten (A) bis (F) eine sehr gute pflegende und kämmbarkeitsverbessernde Wirkung, ohne dabei das Haar zu belasten. Das neue Haarkurmittel wirkt adstringierend und entwirrend auf das Haar, glättet die Haaroberfläche und verbessert den Griff des Haares.

Das erfindungsgemäße Mittel ist zudem leicht biologisch abbaubar, da es keine kationischen Tenside enthält und vorzugsweise keine sonstigen schwer abbaubaren Substanzen, wie zum Beispiel Silicone, enthält.

Das Haarkurmittel ist vorzugsweise von flüssiger bis gelartiger Konsistenz, mit einer Viskosität von etwa 10 bis 2000 mPa·s, insbesondere 20 bis 500 mPa·s, gemessen mit einer Haake-Viskowaage bei 30 Grad Celsius, Stab II und einem Auflagegewicht von 5 g.

Das erfindungsgemäße Mittel ist ohne großen technischen Aufwand kalt herstellbar.

Alle in der vorliegenden Anmeldung angegebenen Prozentzahlen stellen Gewichtsprozente dar.

Folgende Beispiele sollen den Gegenstand der Erfindung näher erläutern:

**Beispiele**

| Beispiel 1: | Haarkurmittel in Gelform |
|---|---|
| 3,0 g | mit 2 Mol Ethylenoxid oxethylierter Laurylalkohol |
| 3,5 g | mit 7 Mol Ethylenoxid oxethylierter Glycerinkokosfettsäuremonoester |
| 1,5 g | kationisches Cellulosederivat nach US-PS 3 472 840 |
| 0,3 g | Zitronensäure, wasserfrei |
| 0,1 g | Parfümöl |
| 28,8 g | Ethanol |
| 62,8 g | Wasser |
| 100,0 g | |

| Beispiel 2: | Haarkurmittel in Gelform |
|---|---|
| 5,00 g | mit 4 Mol Ethylenoxid oxethylierter Laurylalkohol |
| 4,00 g | mit 7 Mol Ethylenoxid oxethylierter Glycerinkokosfettsäuremonoester |
| 1,00 g | kationisches Cellulosederivat nach US-PS 3 472 840 |
| 0,50 g | Zitronensäure, wasserfrei |
| 23,75 g | Ethanol |
| 65,75 g | Wasser |
| 100,0 g | |

| Beispiel 3: | Haarkurmittel in Gelform |
|---|---|
| 6,0 g | mit 4 Mol Ethylenoxid oxethylierter Laurylalkohol |
| 2,5 g | mit 7 Mol Ethylenoxid oxethylierter Glycerinkokosfettsäuremonoester |
| 1,5 g | kationisches Cellulosederivat, zum Beispiel Polymer JR® 400 |
| 0,3 g | Zitronensäure, wasserfrei |
| 0,1 g | Parfümöl |
| 19,0 g | Ethanol |
| 70,6 g | Wasser |
| 100,0 g | |

| Beispiel 4: | Haarkurmittel in flüssiger Form |
|---|---|
| 3,0 g | mit 2 Mol Ethylenoxid oxethylierter Laurylalkohol |
| 3,5 g | mit 7 Mol Ethylenoxid oxethylierter Glycerinkokosfettsäuremonoester |
| 0,1 g | kationisches Cellulosederivat, zum Beispiel Polymer JR® 400 |
| 0,3 g | Zitronensäure, wasserfrei |
| 0,1 g | Parfümöl |
| 30,2 g | Ethanol |
| 62,8 g | Wasser |
| 100,0 g | |

**Vergleichsbeispiele:**

Vergleichsbeispiel A:

Nach einer vorangegangenen Haarwäsche mit einem üblichen, nicht konditionierenden Shampoo wurde das handtuchtrockene, feine, lange und poröse Haar von 4 Versuchspersonen mit einer Haarkur gemäß Beispiel 1 behandelt. Um eindeutige Ergebnisse zu erhalten und von Versuchsperson zu Versuchsperson abweichende Haarqualitäten auszuschalten, wurde das Haar in der Mitte gescheitelt und auf eine Hälfte des Haares, in Abhängigkeit von der Haarfülle, 10 bis 20 g des erfindungsgemäßen Mittels aufgetragen, während die andere Hälfte mit einem Vergleichspräparat I, bestehend aus einer 2prozentigen wäßrigen Lösung von Cetyltrimethylammoniumchlorid, behandelt wurde. Nach einer Einwirkungsdauer von 5 Minuten wurden die Mittel mit warmem Wasser ausgespült und das Haar gekämmt und getrocknet. So konnte die Wirkung der Präparate von einer friseur-fachlichen Expertengruppe sicher beurteilt werden. Eine Bewertung erfolgte nach dem Schema für die zusammengefaßten Kriterien der Naßkämmbarkeit, Glanz, Griff und der statischen Aufladung des Haares.

Benotung:    1 = sehr gut
             2 = gut
             3 = ausreichend
             4 = mangelhaft

Das Ergebnis des Vergleichs beider Mittel zeigt Tabelle 1.

Tabelle 1

| | Note 1 | Note 2 | Note 3 | Note 4 |
|---|---|---|---|---|
| Haarkur gemäß Beispiel 1 (Anzahl der Versuchspersonen) | 3 | 1 | 0 | 0 |
| Vergleichspräparat I (Anzahl der Versuchspersonen) | 1 | 2 | 1 | 0 |

Vergleichsbeispiel B:

Die Haare einer weiteren Gruppe von 4 Versuchspersonen mit langem, feinem und porösem Haar wurden mit einer Haarkur gemäß Beispiel 1 und dem nachstehenden Vergleichspräparat II, entsprechend der im Stand der Technik zitierten JP-OS 65-193 717, in gleicher Weise wie in Vergleichsbeispiel A mit je 15 g der Mittel behandelt.

Vergleichspräparat II (entsprechend JP-OS 65-193 717)

| | |
|---|---|
| 0,3 g | Polyoxyethylensorbitanmonolaurat |
| 0,5 g | kationisches Cellulosederivat, zum Beispiel Polymer JR® 400 |
| 0,5 g | Zitronensäure |
| 20,0 g | Ethanol |
| 78,7 g | Wasser |
| 100,0 g | |

Das Ergebnis des Vergleichs zeigt Tabelle 2.

Tabelle 2

| | Note 1 | Note 2 | Note 3 | Note 4 |
|---|---|---|---|---|
| Haarkur gemäß Beispiel 1 (Anzahl der Versuchspersonen) | 3 | 1 | 0 | 0 |
| Vergleichspräparat II (Anzahl der Versuchspersonen) | 0 | 2 | 2 | 0 |

Das Ergebnis zeigt eine deutlich bessere pflegende Wirkung des erfindungsgemäßen Haarkurmittels, die auf einem synergistischen Effekt der Komponenten (A) bis (F) beruht.

Vergleichsbeispiel C:

Die Haare einer weiteren Gruppe von 4 Versuchspersonen mit langem, feinem Haar wurden mit einer Haarkur gemäß Beispiel 2 und dem nachstehenden, nicht erfindungsgemäßen emulsionsförmigen Vergleichspräparat III in gleicher Weise wie in Vergleichsbeispiel A behandelt.

Vergleichspräparat III

| | |
|---|---|
| 1,50 g | Cetylalkohol |
| 0,49 g | eines Gemisches aus Glycerinmonodistearat, Kaliumstearat, Glycerin und Stearinsäure |
| 0,50 g | Vaseline |
| 0,05 g | mit 25 Mol Ethylenoxid ethoxylierter Cetylstearylalkohol |
| 0,75 g | Cetyltrimethylammoniumchlorid |
| 96,71 g | Wasser |
| 100,00 g | |

Das Ergebnis des Vergleichs beider Mittel zeigt Tabelle 3.

Tabelle 3

| | Note 1 | Note 2 | Note 3 | Note 4 |
|---|---|---|---|---|
| Haarkur gemäß Beispiel 2 (Anzahl der Versuchspersonen) | 2 | 2 | 0 | 0 |
| Vergleichspräparat III (Anzahl der Versuchspersonen) | 0 | 2 | 2 | 0 |

Zudem zeigt das erfindungsgemäße Mittel ein besseres Emulgiervermögen.

Vergleichsbeispiel D:

Feines, langes Haar von 4 Versuchspersonen wurde mit einer 10prozentigen wäßrigen Lösung von Natriumlaurylalkoholdiglykolethersulfat gewaschen, mit warmem Wasser ausgespült und mit dem Handtuch getrocknet. Danach wurde das Haar in der Mitte gescheitelt und auf eine Hälfte des Haares 10 g einer Haarkur gemäß Beispiel 3 aufgebracht. Die andere Hälfte des Haares wurde mit 10 g eines nicht erfindungsgemäßen emulsionsförmigen Präparates IV folgender Zusammensetzung behandelt:

Vergleichspräparat IV

| | |
|---|---|
| 4,00 g | Cetylalkohol |
| 0,49 g | eines Gemisches aus Glycerinmonodistearat, Kaliumstearat, Glycerin und Stearinsäure |
| 0,50 g | Vaseline |
| 0,05 g | mit 25 Mol Ethylenoxid oxethylierter Cetylstearylalkohol |
| 3,00 g | Cetyltrimethylammoniumchlorid |
| 91,96 g | Wasser |
| 100,00 g | |

Anschließend wurden die Haare mit Wasser ausgespült, mit einem Handtuch leicht getrocknet und auf Wasserwellwickler aufgerollt. Nach dem Trocknen der Haare wurden die Wickler entfernt und die Haare toupiert.
Das Ergebnis des Vergleichs beider Mittel zeigt Tabelle 4.

Tabelle 4

| | Note 1 | Note 2 | Note 3 | Note 4 |
|---|---|---|---|---|
| Haarkur gemäß Beispiel 3 (Anzahl der Versuchspersonen) | 2 | 2 | 0 | 0 |
| Vergleichspräparat IV (Anzahl der Versuchspersonen) | 0 | 1 | 3 | 0 |

Das mit der erfindungsgemäßen Haarkur behandelte Haar ist, im Gegensatz zu dem mit Vergleichspräparat IV behandelten Haar, weniger weicher und griffiger und läßt sich leichter zur Frisur toupieren.

**Patentansprüche**

1. Haarkurmittel, dadurch gekennzeichnet, daß es eine Kombination aus den Komponenten

   (A) 0,2 bis 10,0 Gewichtsprozent mindestens eines mit 1 bis 6 Mol Ethylenoxid oxethylierten $C_{12}$- bis $C_{20}$-Fettalkohols

   (B) 0,2 bis 10,0 Gewichtsprozent mindestens eines mit 4 bis 10 Mol Ethylenoxid oxethylierten Glycerylfettsäureesters einer Fettsäure mit 12 bis 20 Kohlenstoffatomen

   (C) 0,01 bis 3,0 Gewichtsprozent mindestens eines kationischen Cellulosederivates,

   (D) 0,01 bis 2,0 Gewichtsprozent mindestens einer wasserlöslichen, physiologisch verträglichen organischen Säure mit 1 bis 10 Kohlenstoffatomen,

   (E) 1,0 bis 40,0 Gewichtsprozent Ethanol und/oder Isopropanol,

   und

   (F) 50 bis 80 Gewichtsprozent Wasser

   enthält und frei von kationischen Tensiden ist.

2. Mittel nach Anspruch 1, dadurch gekennzeichnet, daß es 0,5 bis 7 Gewichtsprozent der Komponente (A) enthält.

3. Mittel nach Anspruch 1 oder 2, dadurch gekennzeichnet, daß es 0,5 bis 7 Gewichtsprozent der Komponente (B) enthält.

4. Mittel nach einem der Ansprüche 1 bis 3, dadurch gekennzeichnet, daß es 0,1 bis 2,0 Gewichtsprozent der Komponente (C) enthält.

5. Mittel nach einem der Ansprüche 1 bis 4, dadurch gekennzeichnet, daß es 0,1 bis 1 Gewichtsprozent der Komponente (D) enthält.

6. Mittel nach einem der Ansprüche 1 bis 5, dadurch gekennzeichnet, daß es 5 bis 35 Gewichtsprozent der Komponente (E) enthält.

7. Mittel nach einem der Ansprüche 1 bis 6, dadurch gekennzeichnet, daß es 55 bis 75 Gewichtsprozent der Komponente (F) enthält.

8. Mittel nach einem der Ansprüche 1 bis 7, dadurch gekennzeichnet, daß es als Komponente (A) mit 2 bis 4 Mol Ethylenoxid oxethylierten Laurylalkohol enthält.

9. Mittel nach einem der Ansprüche 1 bis 8, dadurch gekennzeichnet, daß es als Komponente (B) oxethylierte Glycerylester der Kokosfettsäure und/oder oxethylierte Glycerylmonoester der Laurinsäure enthält.

10. Mittel nach einem der Ansprüche 1 bis 9, dadurch gekennzeichnet, daß es als Komponente (D) Zitronensäure und/oder Milchsäure enthält.

## Claims

1. Hair treatment agent, characterised in that it contains a combination of the components:

(A) 0.2 to 10.0     weight percent of at least one $C_{12}$ to $C_{20}$ fatty alcohol oxyethylated with from 1 to 6 moles of ethylene oxide;

(B) 0.2 to 10.0     weight percent of at least one glyceryl fatty acid ester of a $C_{12}$ to $C_{20}$ fatty acid oxyethylated with from 4 to 10 moles of ethylene oxide;

(C) 0.01 to 3.0     weight percent of at least one cationic cellulose derivative;

(D) 0.01 to 2.0     weight percent of at least one water-soluble, physiologically compatible, $C_1$ to $C_{10}$ organic acid;

(E) 1.0 to 40.0     weight percent ethanol and/or isopropanol;

and

(F) 50 to 80     weight percent of water;

and is free from cationic surfactants.

2. Agent according to Claim 1, characterised in that it contains from 0.5 to 7 weight percent of component (A).

3. Agent according to Claim 1 or 2, characterised in that it contains from 0.5 to 7 weight percent of component (B).

4. Agent according to any one of Claims 1 to 3, characterised in that it contains from 0.1 to 2.0 weight percent of component (C).

5. Agent according to any one of Claims 1 to 4, characterised in that it contains from 0.1 to 1 weight percent of component (D).

6. Agent according to any one of Claims 1 to 5, characterised in that it contains from 5 to 35 weight percent of component (E).

7. Agent according to any one of Claims 1 to 6, characterised in that it contains from 55 to 75 weight percent of component (F).

8. Agent according to any one of Claims 1 to 7, characterised in that it contains lauryl alcohol oxyethylated with from 2 to 4 moles of ethylene oxide as component (A).

9. Agent according to any one of Claims 1 to 8, characterised in that it contains oxyethylated glyceryl esters of coconut fatty acid and/or oxyethylated glyceryl monoesters of lauric acid as component (B).

10. Agent according to any one of Claims 1 to 9, characterised in that it contains citric acid and/or lactic acid as component (D).

**Revendications**

1. Agent de soins capillaires, caractérisé en ce qu'il contient une combinaison des composants:

   (A) de 0,2 à 10,0% en poids d'au moins un alcool gras en $C_{12}$-$C_{20}$ oxy-éthylé avec de 1 à 6 mole d'oxyde d'éthylène,
   (B) de 0,2 à 10,0% en poids d'au moins un ester d'acide gras de la glycérine avec de 12 à 20 atomes de carbone oxy-éthylé avec de 4 à 10 mole d'oxyde d'éthylène,
   (C) de 0,01 à 3,0% en poids d'au moins un dérivé cationique de la cellulose,
   (D) de 0,01 à 2,0% en poids d'au moins un acide organique soluble dans l'eau, physiologiquement compatible, comprenant de 1 à 10 atomes de carbone,
   (E) de 1,0 à 40,0% en poids d'éthanol et/ou d'isopropanol, et
   (F) de 50 à 80% en poids d'eau.

   et se trouve exempt d'agent tensio-actifs cationiques.

2. Agent selon la revendication 1, caractérisé en ce qu'il contient de 0,5 à 7% en poids du composant (A).

3. Agent selon la revendication 1 ou 2, caractérisée en ce qu'il contient de 0,5 à 7% en poids du composant (B).

4. Agent selon l'une des revendications 1 à 3, caractérisé en ce qu'il contient de 0,1 à 2,0% en poids du composant (C).

5. Agent sel on l'une des revendications 1 à 4, caractérisé en ce qu'il contient de 0,1 à 1,0% en poids du composant (D).

6. Agent selon l'une des revendications 1 à 5, caractérisé en ce qu'il contient de 5 à 35% en poids du composant (E).

7. Agent selon l'une des revendications 1 à 6, caractérisé en ce qu'il contient de 55 à 75% en poids du composant (F).

8. Agent selon l'une des revendications 1 à 7, caractérisé en ce qu'il contient en tant que composant (A), de l'alcool laurylique oxy-éthylé avec 4 mole d'oxyde d'éthylène.

9. Agent selon l'une des revendications 1 à 8, caractérisé en ce qu'il contient en tant que composant (B), un ester de glycéryle de l'acide du coprah oxy-éthylé, et/ou un monoester de glycéryle oxy-éthylé de l'acide laurique.

10. Agent sel on l'une des revendications 1 à 9, caractérisé en ce qu'il contient comme composant (D), de l'acide citrique et/ou de l'acide lactique.